# FASCICULE DE BREVET EUROPEEN

(11) **EP 0 618 820 B1**
(45) Date de publication et mention de la délivrance du brevet: **04.03.1998**
(21) Numéro de dépôt: 93918868.6
(22) Date de dépôt: 14.09.1993
(51) Int. Cl.: A61M 5/142, A61M 39/04

(54) **ENSEMBLE CONSTITUANT UNE POMPE A USAGE MEDICAL**
MEDIZINISCHE PUMPENANORDNUNG
MEDICAL PUMP ASSEMBLY

(30) Priorité: 16.09.1992 CH 2919/92
(43) Date de publication de la demande: 12.10.1994
(73) Titulaire: DEBIOTECH S.A., CH-1003 Lausanne (CH)
(72) Inventeur: BROHY, Michel, CH-1802 Corseaux (CH); NEFTEL, Frédéric, CH-1000 Lausanne 9 (CH)
(74) Mandataire: Micheli & Cie
(86) Numéro de dépôt international: CH9300222
(87) Numéro de publication internationale: WO9406491

(56) Documents cités:
- EP-A- 0 111 723
- EP-A- 0 433 485
- EP-A- 0 447 909
- GB-A- 1 077 157
- US-A- 4 684 365
- US-A- 4 685 902

## Description

L'invention a pour objet un ensemble constituant une pompe à usage médical, plus précisément une pompe voire une micropompe portable destinée à l'administration parentérale de solutions médicamenteuses.

On connaît depuis quelques années déjà divers types de pompes ou micropompes à usage médical. Les micropompes dites portables sont particulièrement appréciées dans le traitement thérapeutique de patients tels que diabétiques ou cancéreux par exemple, qui doivent recevoir en permanence, ou pour le moins sur des périodes prolongées, une substance médicamenteuse administrable en continu, à une dose contrôlée. Des pompes ou micropompes dites péristaltiques ont été développées pour un tel usage et sont décrites dans la littérature spécialisée.

La demande de brevet FR-A-89 04044 décrit un tel type de micropompe. Celle-ci comporte deux modules distincts, un module dit "moteur", non stérilisable, comprenant le moteur et son circuit de commande, et un module dit "pompe" proprement dit, stérilisable, comprenant un rotor muni de galets, un tuyau et un presseur coopérant avec les galets pour l'écrasement dudit tuyau. Ces deux modules sont assemblés de façon amovible.

La demande de brevet EP-A-0 447 909 décrit un modèle de micropompe analogue, dont l'une des caractéristiques essentielles consiste en l'assemblage irréversible d'un module "réservoir" et d'un module "moteur". Ce modèle de micropompe comporte en outre divers systèmes de sécurité contribuant à garantir l'asepsie de l'administration de la solution médicamenteuse au patient, de même que toute fausse manoeuvre du seul fait de l'usager. Comme illustré dans EP-A-0 447 909, le module contenant le réservoir destiné à la solution médicamenteuse est muni d'un orifice de remplissage accessible à l'usager lorsque les deux modules sont séparés, mais devenant par la suite inaccessible audit usager lorsque lesdits modules sont assemblés de manière irréversible, selon la caractéristique de cette micropompe. On parvient ainsi à garantir tout nouvel accès à la solution médicamenteuse de même que toute contamination ultérieure de cette dernière, dans la règle stérile au moment du remplissage du réservoir de stockage, ceci durant toute la durée de fonctionnement de la micropompe.

Si l'usage montre que ce type de sécurité remplit parfaitement son rôle une fois la micropompe en fonction, on demeure toujours confronté au problème du maintien de l'asepsie lors du remplissage extemporané du module "réservoir". En effet, le remplissage d'un tel module ne pose pratiquement pas de problème en milieu hospitalier, où l'on dispose soit du personnel spécialisé, soit d'installations adéquates, salle blanche ou hotte à flux laminaire par exemple; par contre, le remplissage par le patient lui-même, par définition une personne non expérimentée, est tout autre lorsqu'il s'agit de maintenir une asepsie des plus rigoureuses lors de cette opération et d'éviter toute contamination accidentelle de la solution médicamenteuse. L'invention apporte une solution nouvelle, originale et techniquement fiable au problème exposé ci-dessus.

On connaît du document EP-A-0.433.485 un dispositif pour le remplissage transcutané de pompes. Ce dispositif comprend, sur une branche de remplissage, un septum destiné à être transpercé par une seringue de remplissage. Le septum est relié par un flexible à un dispositif filtrant et ensuite à une canule. Le dispositif comprend encore une branche de vidange avec un flexible également reliée à la canule.

Le dispositif décrit dans ce document n'est pas destiné à être fixé ou ancré sur la pompe. Il ne comprend donc pas d'élément support facilitant son maniement. La mise en oeuvre du dispositif décrit doit être effectuée par des personnes expérimentées dans les techniques médicales pour obtenir une asepsie adéquate.

Le document EP-A-0.111.723 se réfère à un dispositif pour injecter, par un canal latéral, des solutions médicamenteuses dans un conduit et comprend un embout comportant, à son extrémité libre, un septum et au centre un filtre. Cet embout ne comprend pas d'éléments de support pour être fixé sur un module de pompe et pour maintenir le dispositif filtrant de façon amovible sur cette pompe. L'embout formant le dispositif filtrant est fixé directement sur une entrée latérale du conduit. Le remplissage nécessite donc une certaine dextérité de l'utilisateur qui devra être expérimenté dans les techniques médicales. En outre, l'embout ne pourrait être ôté du conduit après injection, sans cela la solution se perdrait par le canal latéral.

L'invention a pour but de remédier aux inconvénients précités et elle a, à cet effet, plus particulièrement pour objet un ensemble constituant une pompe portable pour l'administration parentérale de solutions médicamenteuses, comprenant un premier module pourvu d'un réservoir de stockage de la solution médicamenteuse, d'un orifice de remplissage dudit réservoir et d'un orifice de sortie amenant ladite solution médicamenteuse à l'extérieur de la pompe, ledit premier module étant agencé pour coopérer avec un second module pourvu d'un moteur et de moyens de pompage, le fonctionnement de la pompe étant assuré par l'assemblage desdits premier et second modules, caractérisé par le fait qu'il comprend un dispositif filtrant stérilisant amovible et un élément de support ayant un bras de support, ce dernier comportant à une première extrémité un organe de fixation destiné à être ancré de façon amovible sur le premier module et, à une seconde extrémité, un organe de maintien destiné à maintenir en place le dispositif filtrant stérilisant amovible de façon que ce dernier soit relié de manière amovible à l'orifice de remplissage.

Dans l'une des exécutions particulières de l'invention, ladite pompe peut être de dimensions fort réduites et ne peser que quelques grammes ou, tout au plus, quelques dizaines de grammes. Dans un tel cas, on parle alors plus volontiers de micropompe; une micropompe selon l'invention convient particulièrement bien au traitement ambulatoire de nombreux types de patients.

Selon l'invention, le dispositif filtrant stérilisant amovible est un dispositif externe; cela revient à dire qu'il se trouve fixé sur la face externe de l'orifice de remplissage. Une fois le remplissage effectué, il peut être facilement ôté du premier module, avant l'assemblage de ce dernier avec le second module.

Dans une exécution particulière de l'invention, l'orifice de remplissage du réservoir est du type septum et le dispositif filtrant stérilisant consiste en une pastille filtrante stérile amovible comportant, d'une part, un embout et, d'autre part, une aiguille passant au travers du septum. Le retrait du dispositif filtrant après usage entraîne bien entendu celui de l'aiguille. Dans une autre exécution de l'invention, l'orifice de remplissage est du type valve anti-retour et le dispositif filtrant stérilisant amovible consiste en une pastille filtrante stérile comportant, d'une part un embout, et d'autre part, un moyen d'assemblage étanche avec la valve anti-retour. Dans un tel cas, seule la pastille filtrante stérile avec son embout est retirée après usage. Des moyens d'assemblage étanche adéquats existent dans le commerce spécialisé.

Selon l'invention, le premier module et le dispositif filtrant stérilisant décrit plus haut sont fabriqués en un matériau résistant à une stérilisation au moyen d'oxyde d'éthylène ou de radiations ionisantes, tels les rayons gamma. De tels matériaux, pour l'essentiel des matériaux polymères, sont bien connus dans le métier.

L'invention a également pour objet un ensemble constituant une pompe portable pour l'administration parentérale de solutions médicamenteuses comprenant un premier module et un second module tels que définis plus haut, dans lequel ledit premier module pourvu du dispositif filtrant stérilisant amovible est présenté séparément du second module, dans une enveloppe étanche en matériaux stérilisables au moyen d'oxyde d'éthylène et/ou de radiations ionisantes.

La mise en oeuvre d'une exécution particulière de l'invention peut être succinctement décrite comme suit : l'enveloppe stérile contenant le module "réservoir" pourvu du dispositif filtrant stérilisant amovible est premièrement ouverte avec précaution. Une seringue standard contenant la solution médicamenteuse, mais dépourvue d'aiguille, est immédiatement connectée à l'embout placé en amont de la pastille filtrante. La solution médicamenteuse est ensuite transférée dans le réservoir de stockage sous la pression exercée à l'aide du piston de la seringue, au travers de la pastille filtrante, après que l'ensemble ait été déplacé de façon à percer le septum. Une fois le transfert de la solution achevé l'usager, tout en maintenant la seringue connectée à l'embout, retire délicatement le dispositif de son emplacement, entraînant de ce fait l'aiguille passée au travers du septum. L'usager connecte ensuite immédiatement le module "moteur" au module "réservoir", la pompe est prête à fonctionner.

Selon un mode d'exécution préféré le dispositif filtrant stérilisant amovible comprend un élément de support comportant une première partie montée de façon amovible sur le premier module et une seconde partie destinée à maintenir en place le dispositif filtrant stérilisant amovible. Cette construction assure une grande sûreté et facilite l'emploi de l'ensemble.

Avantageusement, l'élément de support présente un bras de support muni de ladite première partie constituée par un boîtier d'ancrage agencé de façon à pouvoir être intro2duit dans un logement du premier module et de ladite seconde partie constituée par une pièce de guidage destinée à assurer un coulissement axial du dispositif filtrant stérilisant amovible.

Il est également particulièrement avantageux que l'élément de support comprenne un couvercle destiné à fermer l'ouverture dudit logement et que le dispositif filtrant stérilisant amovible comprenne un manchon de protection élastique reliant le support de l'aiguille à un embout de l'orifice de remplissage. Cette disposition assure à l'ensemble une asepsie améliorée.

De préférence, les moyens de pompage sont du type peristaltique comprenant des tubulaires flexibles disposés dans ledit logement du premier module, le boîtier d'ancrage comportant au moins un organe destiné à maintenir en place les tubulaires. Ainsi, on assure même sous des conditions de transport difficiles, un pompage adéquat ultérieur.

Selon un mode avantageux, l'élément de support présente un logement dans lequel au moins l'aiguille du dispositif filtrant stérilisant amovible peut être disposée après le remplissage du réservoir. L'aiguille est ainsi soigneusement et rapidement emballée avant d'être jetée.

La grande utilité de l'élément de support est particulièrement à souligner, puisque cet élément unique permet de réaliser au moins cinq fonctions importantes, à savoir le support du dispositif filtrant stérilisant amovible facilitant le remplissage du réservoir, le guidage axial de ce dispositif lors du perçage du septum, le remplissage et la fermeture du logement destiné au second module à moteur, le maintien des tubulaires flexibles de la pompe avant son utilisation et finalement, l'emballage pour la mise en sécurité de l'aiguille après usage.

D'autre avantages ressortent des caractéristiques exprimées dans les revendications dépendantes et de la description exposant ci-après l'invention plus en détail à l'aide de dessins qui représentent schématiquement et à titre d'exemple un mode d'exécution et une variante.

Les figures 1a et 1b représentent l'ensemble avant son utilisation.

la figure 2 illustre le premier module lors du remplissage du réservoir.

Les figues 3a et 3b montrent l'ensemble lors de l'administration de la solution médicamenteuse.

La figure 4 est une vue de détail en coupe selon IV-IV de la figure 2.

La figure 5 représente une vue de détail d'une variante.

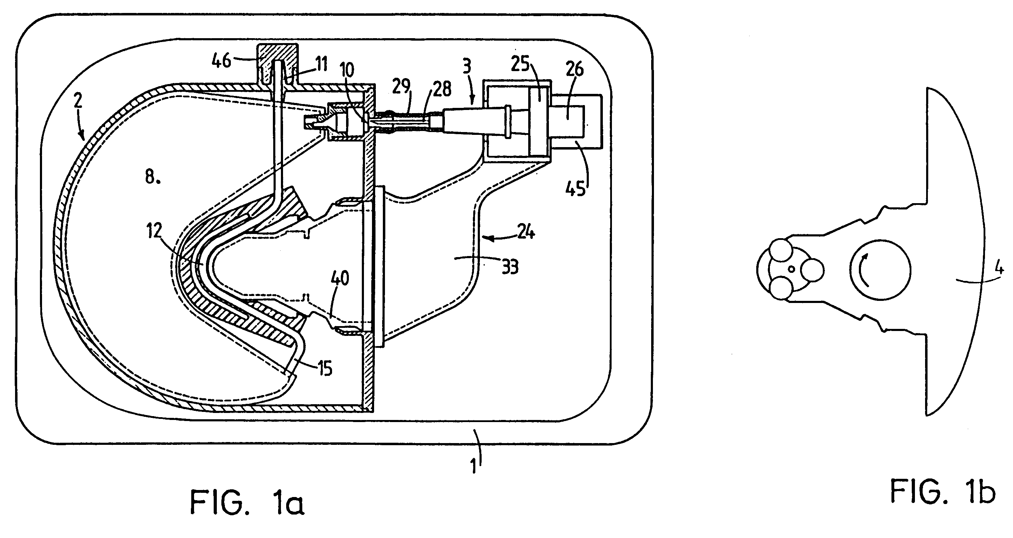

En référence aux figures 1a et 1b, l'ensemble est présenté avant son utilisation sous forme d'un emballage 1 hermétiquement fermé constituant une enveloppe stérile étanche qui contient un premier module 2 avec un dispositif filtrant stérilisant amovible 3 et d'un second module 4 séparé, pourvu d'un moteur. Le premier module 2 et son emballage sont entièrement constitués de matériaux stérilisables, au moyen soit d'oxyde d'éthylène, soit de radiations ionisantes, tels les rayons gamma, tandis que le second module 4 n'est en principe pas à stériliser.

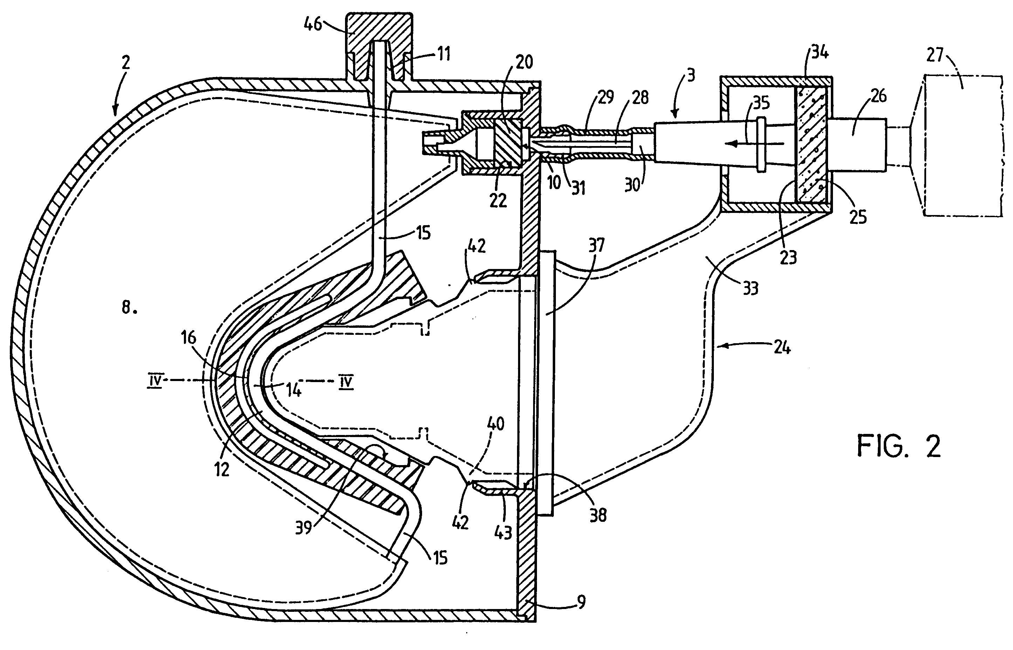

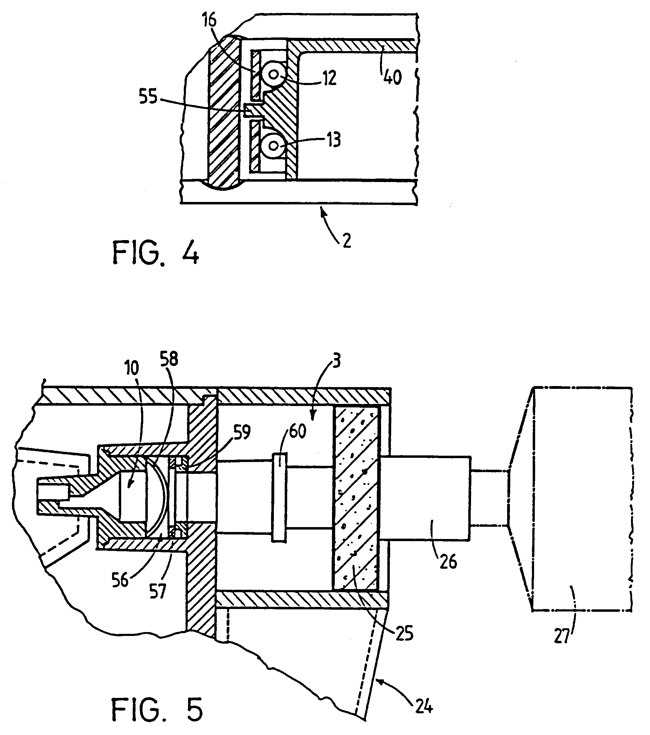

Avant l'administration de la solution médicamenteuse, il est nécessaire de remplir le réservoir de stockage 8 du premier module 2. Ce module est illustré plus en détail à la figure 2 et comprend une paroi extérieure 9 contenant le réservoir 8 relié à un orifice de remplissage 10 et à un orifice de sortie 11. Un tuyau 15 faisant partie d'une pompe péristaltique amène la solution médicamenteuse du réservoir 8 à l'orifice de sortie 11. Ce tuyau 15 se divise en deux tubulaires élémentaires 12,13 (voir fig. 4) dans sa partie médiane 14 où ces tubulaires élémentaires 12,13 s'appuient d'une part sur un support de pompe 16 et coopèrent d'autre part avec des galets du second module 4 à moteur lorsque ce second module 4 est monté sur le premier module 2 de façon à constituer la pompe péristaltique qui est par ailleurs décrite de façon détaillée dans la demande EP-A-0.447.909.

L'orifice de remplissage 10 est réalisé de préférence comme un septum 20 comportant une pastille en élastomère contenue dans une enceinte 22. Le dispositif filtrant stérilisant amovible 3 proprement dit est fixé sur la face externe de l'orifice de remplissage 10 et composé d'un élément de support 24, d'une pastille filtrante stérile usuelle 25 ayant une porosité de 0,22 micron par exemple, contenu dans un boîtier 23 ayant un embout médical standard 26 pour le raccordement à une seringue usuelle 27, d'une aiguille médicale conventionnelle 28 et d'un manchon de protection élastique 29 collé sur le support aiguille 30 et s'adaptant fermement sur l'embout 31 de l'orifice 10.

L'élément de support 24 présente une forme complexe et comporte une première partie constituée par un bras de support 33 muni à l'une de ses extrémités d'une pièce de guidage 34 destinée à assurer un coulissement du boîtier 23 suivant une direction axiale 35.

A son autre extrémité le bras de support comporte un couvercle 37 destiné à fermer l'ouverture 38 du logement 39 dans lequel le second module 4 devra être introduit lors de l'administration de la solution médicamenteuse. Le bras de support 33 avec son couvercle 37 est prolongé par un boîtier 40 ayant une forme similaire au second module 4. Ce boîtier 40 est introduit dans le logement 39 et retenu par des saillies 42 coopérant avec des lames flexibles 43 venues d'une pièce avec les parois 9 du premier module 2. Ainsi, le boîtier 40 constitue un moyen d'ancrage stable de l'élément support 24 sur le premier module 2. Tel que visible à la figure 1 l'embout 26 et l'orifice de sortie 11 sont munis de bouchons 45,46 fermant le premier module 2. Ces bouchons pourront être étanches ou non en fonction du mode de stérilisation retenu, au moyen de radiations ionisantes ou d'oxyde d'éthylène.

Lorsqu'on désire utiliser l'ensemble, l'enveloppe stérile 1 est ouverte avec précaution et les bouchons 45,46 sont ôtés de l'embout 26 et de l'orifice de sortie 11. Un cathéter 52 est relié à l'orifice de sortie 11 et une seringue standard contenant la solution médicamenteuse, mais dépourvue d'aiguille, est immédiatement connectée à l'embout 26 placé en amont de la pastille filtrante 25. La seringue 27, la pastille filtrante 25 et l'aiguille 28 sont ensuite déplacées axialement vers la gauche à la figure 2 de façon que l'aiguille 28 traverse le septum 20. Lorsque le support aiguille 30 entre en contact avec l'embout 31, le septum 20 est entièrement traversé et la solution médicamenteuse peut être transférée dans le réservoir 8 au travers de la pastille filtrante 25 sous la pression exercée à l'aide du piston de la seringue. L'élément de support 24 avec sa pièce de guidage 34 assure une grande stabilité de l'ensemble et un bon coulissement de la pastille filtrante 25 et de l'aiguille pendant son introduction, lors de laquelle le manchon de protection élastique 29 est comprimé.

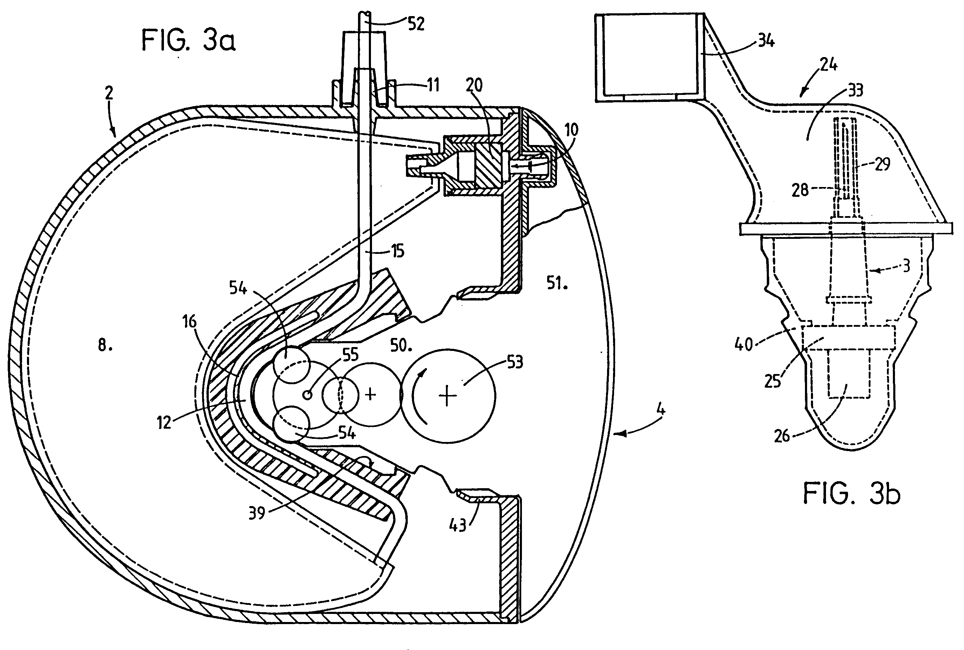

Les figures 3a et 3b illustrent la situation après le remplissage du réservoir 8. Une fois le transfert de la solution médicamenteuse achevé, l'usager, tout en maintenant la seringue 28 connectée à l'embout 26, retire la totalité de l'élément de support 24 avec le boîtier 40, en vainquant la force de retenue constituée par les lames flexibles 43. Ce retrait entraîne bien entendu celui de l'aiguille 28 et du manchon de protection 29 par déchaussement de l'embout 31. L'usager connecte ensuite immédiatement le second module 4 à moteur au premier module 2 en introduisant sa partie proéminente 50 dans le logement 39. La partie extérieure 51 de ce second module 4 est agencée de façon à fermer complètement l'orifice de remplissage 10 pour garantir une bonne asepsie de l'ensemble. Le cathéter 52 connecté à l'orifice de sortie 11, la solution médicamenteuse est amenée à débit contrôlé vers le patient.

A cet effet, le moteur 53 contenu dans le second module 4 entraîne par l'intermédiaire d'engrenages un rotor muni de galets 54 en rotation autour de l'axe 55. Ces galets 54 coopèrent avec les tubulaires élémentaires élastiques 12 et 13 pour constituer une pompe de type péristaltique.

La seringue 27 pourra alors être enlevée de l'embout 26. L'ensemble constitué par la pastille filtrante 25, l'aiguille 28 et le manchon 29 sera ensuite retiré de la pièce de guidage 34 et disposé à l'intérieur du boîtier 40 et du bras de support 33 qui comporte à cet effet une face ouverte ou partiellement ouverte. L'élément de support 24 constitue donc à la fois des moyens pour maintenir le dispositif filtrant stérilisant amovible 3 en place lors du remplissage du réservoir 8 et des moyens de protection de l'aiguille à jeter.

La figure 4 montre que l'élément de support avec son boîtier 40 possède une troisième fonction importante, qui est le maintien des deux tubulaires élémentaires élastiques 12,13 de la pompe péristaltique avant l'introduction du second module 4 à moteur. En effet, ces deux tubulaires élémentaires 12,13 pourraient se déplacer latéralement lors du transport à l'intérieur du premier module et ainsi empêcher un fonctionnement correcte de la pompe péristaltique lors de l'administration de la solution médicamenteuse. Pour empêcher de tels déplacements indésirables le boîtier 40 est muni dans sa partie frontale d'au moins une protubérance 55 destinée à maintenir les tubulaires élémentaires 12,13 en place, lorsque l'élément de support 24 est monté sur le premier module 2.

Selon une variante illustrée à la figure 5, l'orifice de remplissage 10, au lieu de comporter un septum, est muni d'une valve anti-retour 56. Cette dernière présente un clapet 57 sollicité élastiquement par des lamelles élastiques 58 contre un siège 59. L'élément de support 24 maintient comme précédemment décrit de façon amovible une pastille filtrante stérile 25 comportant d'une part un embout 26 pour une seringue 27 et d'autre part un organe d'assemblage étanche 60 avec la valve anti-retour 56. Il est bien entendu que la pastille filtrante stérile 25 est ici maintenue de façon rigide sans coulissement par l'élément de support 24.

Le premier module 2 et les dispositifs filtrants stérilisants décrits ci-dessus sont fabriqués dans des matériaux résistant à une stérilisation au moyen d'oxyde d'éthylène et/ou de radiations ionisantes tels les rayons gamma. De tels matériaux, pour l'essentiel des matériaux polymères, sont bien connus de l'homme du métier. La construction de l'ensemble permet de réaliser des pompes de très petite taille ou micropompes.

Il est bien entendu que les modes de réalisation décrits ci-dessus ne présente aucun caractère limitatif et qu'ils peuvent recevoir toutes modifications désirables à l'intérieur du cadre tel que défini par la revendication 1. En particulier, la pompe pourra être réalisée de façon différente ou être d'une autre type. Des moyens de commande plus ou moins perfectionnés pourront être associés à la pompe, permettant une administration médicamenteuse contrôlée dans le temps et/ou obéissant par exemple à des paramètres physiologiques mesurés. La forme et la constitution de l'élément de support et du réservoir pourront être réalisées différemment, ainsi le bras de support 33 pourra présenter une forme similaire à la partie extérieure 51 du second module 4 à moteur.

## Revendications

1. Ensemble constituant une pompe portable pour l'administration parentérale de solutions médicamenteuses, comprenant un premier module (2) pourvu d'un réservoir de stockage (8) de la solution médicamenteuse, d'un orifice de remplissage (10) dudit réservoir et d'un orifice de sortie (11) amenant ladite solution médicamenteuse à l'extérieur de la pompe, ledit premier module (2) étant agencé pour coopérer avec un second module (4) pourvu d'un moteur et de moyens de pompage, le fonctionnement de la pompe étant assuré par l'assemblage desdits premier et second modules, caractérisé par le fait qu'il comprend un dispositif filtrant stérilisant amovible (3) et un élément de support (24) ayant un bras de support (33), ce dernier comportant à une première extrémité un organe de fixation (40) destiné à être ancré de façon amovible sur le premier module (2) et, à une seconde extrémité, un organe de maintien (34) destiné à maintenir en place le dispositif filtrant stérilisant amovible (3) de façon que ce dernier soit relié de manière amovible à l'orifice de remplissage (10).

2. Ensemble selon la revendication 1, caractérisé en ce que l'organe de fixation est constitué par un boîtier d'ancrage (40) agencé de façon à pouvoir être introduit dans un logement (39) du premier module, ce logement étant destiné à recevoir une partie proéminente (50) du second module (4), lors de son assemblage sur le premier module (2).

3. Ensemble selon la revendication 2, caractérisé en ce que l'élément de support (24) comprend un couvercle (37) destiné à fermer l'ouverture (38) dudit logement (39).

4. Ensemble selon la revendication 2 ou 3, caractérisé en ce que les moyens de pompage sont du type péristaltique comprenant des tubulaires flexibles (12,13) disposés dans ledit logement (39) du premier module (2), le boîtier d'ancrage (40) comportant au moins un organe (55) destiné à maintenir en place les tubulaires (12,13).

5. Ensemble selon l'une des revendications 1 à 4, caractérisé en ce que l'orifice de remplissage (10) est du type septum (20) et le dispositif filtrant stérilisant amovible (3) consiste en une pastille filtrante stérile (25) comportant, d'une part, un embout (26) et, d'autre part, une aiguille (28) destinée à traverser le septum (20), et en ce que ledit organe de maintien est constitué par une pièce de guidage (34) destinée à assurer un coulissement axial du dispositif filtrant stérilisant amovible (3) pour le perçage du septum (20) par l'aiguille (28).

6. Ensemble selon la revendication 5, caractérisé en ce que le dispositif filtrant stérilisant amovible (3) comprend un support (30) pour l'aiguille (28) et un manchon de protection élastique (29) reliant le support (30) à un embout (31) de l'orifice de remplissage (10).

7. Ensemble selon la revendication 5 ou 6, caractérisé en ce que l'élément de support (24) présente un logement dans lequel au moins l'aiguille (28) du dispositif filtrant stérilisant amovible (3), ou ce dernier en entier, peut être disposé après le remplissage du réservoir (8).

8. Ensemble selon l'une des revendications 1 à 4, caractérisé en ce que l'orifice de remplissage est du type valve anti-retour (56) et le dispositif filtrant stérilisant amovible (3) consiste en une pastille filtrante stérile (25) comportant, d'une part, un embout (26) et, d'autre part, un moyen d'assemblage étanche (60) avec la valve anti-retour (56).

9. Ensemble selon l'une des revendications 1 à 8, caractérisé en ce que le premier module (2) et le dispositif filtrant stérilisant amovible (3) sont en matériaux stérilisables au moyen d'oxyde d'éthylène et/ou de radiations ionisantes.

10. Ensemble selon l'une des revendications 1 à 9, caractérisé en ce que le premier module (2) pourvu du dispositif filtrant stérilisant amovible (3) est présenté séparément du second module (4) dans une enveloppe étanche (1) en un matériau stérilisable au moyen d'oxyde d'éthylène et/ou de radiations ionisantes.

11. Ensemble selon la revendication 1, caractérisé en ce qu'une fois le dispositif filtrant stérilisant amovible et l'élément support (24) ôtés de leur position initiale après le remplissage du réservoir (8) de stockage, ledit orifice de remplissage (10) est rendu inaccessible à l'usager par l'assemblage des premier (2) et second (4) modules.

## Claims

1. A portable medical pump assembly for the parenteral administration of medicinal solutions, comprising a first module (2) provided with a storage reservoir (8) for the medicinal solution, an inlet (10) for said reservoir and an outlet (11) to take said medicinal solution outside the pump, said first module (2) being arranged to cooperate with a second module (4) provided with a motor and pumping means, the functioning of the pump being provided for by the assembly of said first and second modules, characterized in that it comprises a removable sterilizing filtering device (3) and a support member (24) having a support arm (33), the latter having at a first end a fastening member (40) designed for anchoring removably on the first module (2) and, at a second end, a retention member (34) designed for retaining in place the removable sterilizing filtering device (3) such that the latter be connected removably to the filling inlet (10).

2. An assembly according to claim 1, characterized in that the fastening member is comprised of an anchoring housing (40) arranged so that it can be introduced into a housing (39) of the first module, this housing being designed for receiving a protruding part (50) of the second module (4) upon its assembling on the first module (2),

3. An assembly according to claim 2, characterized in that the support member (24) comprises a cover (37) designed for closing the opening (38) of said housing (39).

4. An assembly according to claim 2 or 3, characterized in that the pumping means are of the peristaltic type including flexible tubings (12, 13) arranged in said housing (39) of the first module (2), the anchoring housing (40) including at least one member (55) designed for maintaining in place the tubings (12, 13).

5. An assembly according to one of claims 1 to 4, characterized in that the filling inlet (10) is of the septum type (20) and the removable sterilizing filtering device (3) consists of a sterile filtering pellet (25) including, on the one hand, a connector (26) and, on the other hand, a needle (28) designed for traversing the septum (20) and in that the retaining member is comprised of a guiding piece (34) designed for ensuring an axial sliding of the removable sterilizing filtering device (3) for the piercing of the septum (20) by the needle (28).

6. An assembly according to claim 5, characterized in that the removable sterilizing filtering device (3) comprises a support (30) for the needle (28) and a resilient protective sleeve (29) connecting the support (30) to a connector (31) of the filling port (10).

7. An assembly according to claim 5 or 6, characterized in that the support member (24) is provided with a housing in which at least one needle (28) of the removable sterilizing filtering device (3) or the latter in its entirety, can be disposed after the filling of the reservoir (8).

8. An assembly according to one of claims 1 to 4, characterized in that the filling port is of the nonreturn valve type (56) and the removable sterilizing filtering device (3) consists of a sterile filtering pellet (25) carrying, on the one hand, a connector (26) and, on the other hand, a watertight assembling means (60) with the non return valve (56).

9. An assembly according to one of claims 1 to 8, characterized in that the first module (2) and the removable sterilizing filtering device (3) are made of materials which can be sterilized by means of ethylene oxide and/or ionising radiations.

10. An assembly according to one of claims 1 to 9, characterized in that the first module (2) provided with a removable sterilizing filtering device (3) is provided separately from the second module (4) in a sealed envelope (1) of a material which can be sterilized by means of ethylene oxide and/or of ionising radiations.

11. An assembly according to claim 1, characterized in that once the removable sterilizing filtering device and the support element (24) are removed from their initial position after the filling of the storage reservoir (8), said filling inlet (10) is made non accessible to the user by the assembling of the first (2) and of the second (4) modules.

## Patentansprüche

1. Tragbare Pumpenanordnung für die parenterale Verabreichung von Arzneimittellösungen mit einem ersten Modul (2), der mit einem Vorratsbehälter (8) für die Arzneimittellösung, einem Füllloch (10) für diesen Behälter und einer Austrittsöffnung (11), die die benannte Arzneimittellösung aus der Pumpe herausfördert, ausgestattet ist, wobei der benannte erste Modul (2) dafür eingerichtet ist, mit einem zweiten Modul (4) zusammenzuwirken, der mit einem Motor und Pumporganen ausgestattet ist, und der Betrieb der Pumpe durch das Zusammenfügen des benannten ersten und zweiten Moduls gewährleistet ist, dadurch gekennzeichnet, dass sie eine abnehmbare, sterilfiltrierende Vorrichtung (3) und ein Stützelement (24) mit Stützarm (33) umfasst, wobei dieser Arm an einem ersten Ende ein Befestigungsorgan (40) hat, das dazu bestimmt ist, abnehmbar auf dem ersten Modul (2) verankert zu werden, während er an einem zweiten Ende ein Halteorgan (34) hat, das dazu bestimmt ist, die abnehmbare, sterilfiltrierende Vorrichtung (3) so an Ort und Stelle zu halten, dass diese Vorrichtung abnehmbar an das Füllloch (10) angeschlossen ist.

2. Anordnung gemäss Anspruch 1, dadurch gekennzeichnet, dass das Befestigungsorgan aus einem Verankerungsgehäuse (40) besteht, das so eingerichtet ist, dass es in einen Lagersitz (39) des ersten Moduls eingeführt werden kann, wobei dieser Lagersitz dazu bestimmt ist, eine vorstehende Partie (50) des zweiten Moduls (4) aufzunehmen, wenn dieser mit dem ersten Modul (2) zusammengefügt wird.

3. Anordnung gemäss Anspruch 2, dadurch gekennzeichnet, dass das Stützelement (24) einen Deckel (37) umfasst, der dazu bestimmt ist, die Öffnung (38) des benannten Lagersitzes (39) zu verschliessen.

4. Anordnung gemäss Anspruch 2 oder 3, dadurch gekennzeichnet, dass die Pumporgane vom peristaltischen Typ sind und Gummischläuche (12, 13) umfassen, die in dem benannten Lagersitz (39) des ersten Moduls (2) angeordnet sind, wobei das Verankerungsgehäuse (40) zumindest ein Organ (55) hat, das dazu bestimmt ist, die Schläuche (12, 13) an Ort und Stelle zu halten.

5. Anordnung gemäss einem der Ansprüche 1 bis 4, dadurch gekennzeichnet, dass das Füllloch (10) von der Art eines Septums (20) ist und die abnehmbare, sterilfiltrierende Vorrichtung (3) aus einem sterilfiltrierenden Plättchen (25) besteht, das einerseits einen Stutzen (26) und andererseits eine Nadel (28) umfasst, die dazu bestimmt ist, durch das Septum (20) hindurchzugehen, und dadurch, dass das benannte Halteorgan aus einem Führungsteil (34) besteht, das dazu bestimmt ist, ein axiales Gleiten der abnehmbaren, sterilfiltrierenden Vorrichtung (3) zum Durchstechen des Septums (20) durch die Nadel (28) zu gewährleisten.

6. Anordnung gemäss Anspruch 5, dadurch gekennzeichnet, dass die abnehmbare, sterilfiltrierende Vorrichtung (3) eine Stütze (30) für die Nadel (28) und eine elastische Schutzhülle (29) umfasst, die die Stütze (30) mit einem Stutzen (31) des Fülllochs (10) verbindet.

7. Anordnung gemäss Anspruch 5 oder 6, dadurch gekennzeichnet, dass das Stützelement (24) eine Tasche aufweist, worin zumindest die Nadel (28) der abnehmbaren, sterilfiltrierenden Vorrichtung (3) oder aber diese Vorrichtung als Ganzes nach der Füllung des Behälters (8) entsorgt werden kann.

8. Anordnung gemäss einem der Ansprüche 1 bis 4, dadurch gekennzeichnet, dass das Füllloch vom Typ eines Rückschlagventils (56) ist und die abnehmbare, sterilfiltrierende Vorrichtung (3) aus einem sterilfiltrierenden Plättchen (25) besteht, das einerseits einen Stutzen (26) und andererseits ein Organ (60) zum dichten Zusammenfügen mit dem Rückschlagventil (56) umfasst.

9. Anordnung gemäss einem der Ansprüche 1 bis 8, dadurch gekennzeichnet, dass der erste Modul (2) und die abnehmbare, sterilfiltrierende Vorrichtung (3) aus Materialien bestehen, die mit Hilfe von Ethylenoxid und/oder ionisierender Strahlung sterilisiert werden können.

10. Anordnung gemäss einem der Ansprüche 1 bis 9, dadurch gekennzeichnet, dass der erste, mit der abnehmbaren, sterilfiltrierenden Vorrichtung (3) ausgestattete Modul (2) getrennt vom zweiten Modul (4) in einer dichten Hülle (1) aus mit Hilfe von Ethylenoxid und/oder ionisierender Strahlung sterilisierbarem Material verpackt ist.

11. Anordnung gemäss Anspruch 1, dadurch gekennzeichnet, dass das benannte Füllloch (10) durch das Zusammenfügen des ersten (2) und zweiten (4) Moduls für den Benutzer unzugänglich gemacht wird, nachdem die abnehmbare, sterilfiltrierende Vorrichtung und das Stützelement (24) nach Füllung des Vorratsbehälters (8) aus ihrer anfänglichen Lage entfernt worden sind.
